# FASCICULE DE BREVET EUROPEEN

(11) **EP 2 224 900 B1**
(45) Date de publication et mention de la délivrance du brevet: **12.07.2017**
(21) Numéro de dépôt: 08871824.2
(22) Date de dépôt: 27.11.2008
(51) Int. Cl.: A61K 8/19, A61K 8/49, A61Q 19/08, A61K 41/00

(54) **Composition pour le traitement photodynamique de la peau**
Zusammensetzung zur photodynamischen Hautbehandlung
Composition for photodynamic skin treatment

(30) Priorité: 27.11.2007 FR 0708284
(43) Date de publication de la demande: 08.09.2010
(73) Titulaire: Eymard Du Vernet, Michèle, 75017 Paris (FR)
(72) Inventeur: Eymard Du Vernet, Michèle, 75017 Paris (FR)
(74) Mandataire: L'Helgoualch, Jean
(86) Numéro de dépôt international: PCT/FR2008/001650
(87) Numéro de publication internationale: WO 2009/095568

(56) Documents cités:
- EP-A- 0 584 552
- EP-A- 1 834 955
- EP-A2- 0 515 768
- WO-A-01/40232
- WO-A-94/02484
- WO-A-03/039597
- WO-A-03/047533
- US-A1- 2007 148 222

## Description

La présente invention concerne une composition dermatologique à base de chlorophylle et de chlorophylline, et leurs dérivés, pour le traitement et la prévention des signes du vieillissement cutané, de l'héliodermite et de la Kératase actipique par la technique de la thérapie photodynamique

La peau constitue à elle seule un organe qui remplit plusieurs fonctions déterminantes sur la santé de l'être humain ou animal. Elle constitue une barrière vis-à-vis de l'environnement et la qualité de ses défenses vis-à-vis des agressions de l'environnement est donc un élément majeur pour l'efficacité de sa protection.

La peau comprend plusieurs couches intégrées, à savoir une couche superficielle constituée par l'épiderme, et deux couches plus profondes formant le derme et l'hypoderme. Chacune de ces couches possède des propriétés spécifiques permettant à l'ensemble de réagir et de s'adapter aux conditions de son environnement.

L'épiderme est principalement composé de kératinocytes (90% des cellules épidermiques), de mélanocytes (2 à 3% des cellules épidermiques) et des cellules de Langerhans. Son épaisseur est variable selon les différentes parties du corps. Le derme est plus épais, et se compose principalement de collagène, d'élastine et de protéoglycanes. Ces trois types de molécules sont synthétisés par les fibroblastes dermiques. Les fibres de collagène assurent la résistance mécanique et la texture de la peau, l'élastine est responsable de l'élasticité, et les protéoglycanes jouent un rôle majeur de structure et d'hydratation de la peau. D'autres cellules comme les macrophages et les leucocytes sont également présentes dans la couche du derme. L'hypoderme est la couche la plus profonde de la peau, et contient les adipocytes qui produisent des lipides afin que le tissu sous-cutané puisse fabriquer une couche grasse protégeant les muscles, les os et les organes internes contre les chocs.

Le système immunitaire cutané regroupe un grand nombre d'acteurs cellulaires tels que les kératinocytes, les cellules de Langerhans, les fibroblastes, les mastocytes, les macrophages et les cellules endothéliales, ainsi que des leucocytes, et divers médiateurs inflammatoires solubles tels que cytokines et chimiokines. Ce système immunitaire cutané assure le maintien de l'homéostasie cutanée. Il est aussi responsable de l'activation et de la régulation des réactions inflammatoires normales et pathologiques.

Le vieillissement de la peau peut être intrinsèque, ou extrinsèque c'est-à-dire provoqué par l'environnement, y compris les agressions climatiques, qui peuvent notamment contribuer à accélérer la dégradation du collagène du derme, et en particulier l'exposition au soleil, les variations de températures et les radicaux libres. Les premiers signes du vieillissement de la peau, tels que les rides et ridules, sont généralement provoqués par le stress et les changements biologiques et physiologiques, accélérés par l'environnement extérieur ou par les modes de vie. L'apparition de marques pigmentaires, la diminution de l'épaisseur de la peau et son affaissement sont également des changements observés au cours du vieillissement. Un sait que la capacité de la peau à remplacer le collagène endommagé diminue avec le temps, et en conséquence des espaces et des irrégularités apparaissent dans le réseau du collagène.

Divers traitements ont été envisagés pour tenter de limiter la dégradation de la peau sous l'effet du vieillissement, voire de provoquer le rajeunissement des cellules cutanées pour améliorer l'apparence et l'état de la peau.

Ainsi, on a proposé des traitements au moyen de compositions sous forme de sérums, crèmes et lotions contenant des alpha-hydroxy acides ou des rétinoïdes, appliquées régulièrement pour réduire progressivement le nombre de rides et ridules, ou encore des compositions topiques à base de pentapeptides du type Lys-Thr-Thr-Lys-Ser, favorisant la synthèse du collagène et des glycosaminoglycannes et procurant un effet sur la régénération cutanée comme décrit dans le brevet FR-A-2.783.169. On a aussi proposé des implants de collagène et d'acide hyaluronique pour dissimuler les lignes d'expression autour des yeux ou de la bouche, la dermabrasion et les peelings chimiques pour éliminer la couche supérieure de la peau endommagée, la chirurgie esthétique, telle que la blépharoplastie (chirurgie des paupières) ou un lifting pour retendre une peau présentant un affaissement, ou encore une restructuration à l'aide d'un laser au dioxyde de carbone pour éliminer les ridules.

Des études ont montré que des traitements utilisant la technique de thérapie photodynamique (PDT) peuvent aussi être utiles. En effet, on sait que certaines substances possèdent des propriétés photosensibilisantes et peuvent être utilisées en thérapie photodynamique pour le traitement et le diagnostic de certaines maladies. Par exemple, des techniques de thérapie photodynamique ont été proposées en dermatologie pour le traitement de kératoses actiniques, du psoriasis et du vitiligo, en ophtalmologie pour le traitement de la dégénérescence maculaire liée à l'âge, ainsi qu'en cancérologie pour le traitement de tumeurs cérébrales, de tumeurs de l'appareil digestif, du poumon ou de la prostate.

La technique de la PDT consiste à administrer la substance photosensibilisante, qui se fixe préférentiellement dans les tissus, par exemple sur les cellules tumorales, et à irradier ensuite les tissus au moyen d'une source lumineuse de longueur d'onde appropriée, susceptible d'activer la substance photosensibilisante. La substance photosensibilisante, activée par une lumière monochromatique (laser ou diode électroluminescente) ou polychromatique (lampe), passe de son état initial d'énergie de base à un état d'énergie supérieur, et peut libérer in situ de l'oxygène singulet ou des radicaux libres qui sont extrêmement réactifs et oxydent les tissus immédiatement voisins, provoquant la mort des cellules, et en particulier des cellules cancéreuses. Ces techniques sont décrites notamment par K.R. Weishaupt et al., Cancer Research, p. 2326-2329 (1976) et T.J. Dougherty et al. "Photodynamic therapy (PDT) and clinical application", Biomédical Photonics Handbook. CRC Press LLC. 38: 1-38 (2003). Les espèces radicalaires oxygénées ainsi produites ont généralement une faible diffusion et une très courte durée de vie si bien que leur effet toxique est très localisé.

Les premières substances étudiées dans ce domaine sont des dérivés d'hématoporphyrine tels que le porfimère sodique (Photofrin®) qui est un mélange d'esters et d'éthers de dihématoporphyrine. Le Photofrin® présente cependant l'inconvénient de devoir être administré par voie intraveineuse deux jours avant le traitement d'activation par la source lumineuse, et son accumulation dans l'organisme avant élimination entraîne une photosensibilisation cutanée pendant 4 à 8 semaines ; de plus, à la longueur d'onde utilisée (630 nm) la pénétration de la lumière est limitée à quelques millimètres, et l'utilisation de cet agent photosensibilisant est donc limitée aux tumeurs de petite dimension, localisées en surface des tissus.

De nombreux autres dérivés ont été proposés pour les traitements par PDT, par exemple des dérivés de benzoporphyrine qui ont pour avantage d'être activés à des longueurs d'ondes plus élevées que le Photofrin (env. 690 nm), ce qui a pour conséquence une meilleure pénétration des rayons lumineux dans les tissus. De plus les dérivés de behzoporphyrine sont éliminés plus rapidement de l'organisme et présentent moins d'effets secondaires que le Photofrin®, en particulier une moindre photosensibilisation cutanée. Un médicament à base d'une benzoporphyrine, le verteporfin (Visudyne®), est utilisé pour le traitement de la dégénérescence maculaire liée à l'âge. Le brevet EP 1834955 décrit des dérivés de porphyrine utilisables en PDT anticancéreuse comme photosensibilisateurs.

On a aussi proposé d'utiliser l'effet photosensibilisant de l'acide aminolévulinique (ALA) pour le traitement des cellules de l'épiderme comme indiqué par J.S. Dover et al., Archives of Dermatology, vol. 141, P. 1247-1252 (Oct. 2005). L'acide aminolévulinique agit comme précurseur de la protoporphyrine IX, et a pour effet, après activation par la lumière à une longueur d'onde d'environ 630 nm, de détruire des cellules épidermiques et de provoquer un effet subséquent de photorajeunissement. L'ester méthylique de l'acide aminolévulinique (Metvix®) présente une meilleure biodisponibilité et est proposé dans le traitement de kératoses actiniques et de carcinomes superficiels. On sait cependant que le taux de pénétration de l'acide aminolévulinique à travers la peau est relativement faible, même sous forme d'ester, ce qui limite sa biodisponibilité. De plus, l'utilisation de l'acide aminolévulinique peut entraîner l'apparition d'érythèmes selon les doses utilisées.

Plus récemment, on a montré que des dérivés de bactériochlorophylle, comme par exemple ceux décrits dans le brevet EP 584.552, possèdent d'intéressantes propriétés photosensibilisantcs utilisables en thérapie photodynamique.

L'utilisation de composés à noyau porphinique extraits de plantes chlorophylliennes, tels que chlorophylle et phéophytine, dans des compositions cosmétiques ayant des effets de protection et de restructuration de la peau, a été décrit dans la demande de brevet WO 94002484. D'autres dérivés de bactériochlorphylle utilisables en thérapie anticancéreuse par PDT sont décrits dans la demande de brevet WO 0140232.

Cependant, on ne connaît pas de substance photosensibilisante utilisable en dermatologie par la technique de la thérapie photodynamique pour traiter efficacement les signes du vieillissement cutané.

Il est donc souhaitable de pouvoir disposer de nouvelles compositions adaptées au traitement dermatologique ou cosmétique de la peau par la technique de la thérapie photodynamique (PDT).

Les études effectuées par la demanderesse ont montré qu'il est possible d'utiliser efficacement la chlorophylle et certains de ses dérivés, comme substance photosensibilisante pour des traitements par PDT, afin de traiter les signes du vieillissement cutané ou de provoquer le rajeunissement de la peau, et que cette substance photosensibilisante pouvait être avantageusement combinée, au sein de la même composition, à certains autres principes actifs ayant une activité complémentaire.

Les études effectuées par la demanderesse ont aussi montré que certains dérivés de chlorophylle, en particulier la chlorophylline, jouent un rôle protecteur limitant les effets sedondaires de l'acide aminolévulinique, en évitant notamment l'apparition d'érythème, et favorisant son efficacité.

La présente invention a pour objet une composition dermatologique sous forme de gel ou d'hydrogel réticulé, comprenant comme substance photosensibilisante de la chlorophylle ou un dérivé représentés par la formule générale (I) : 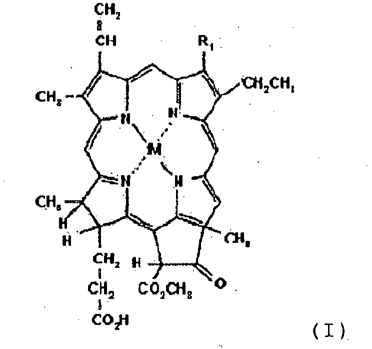 dans laquelle M représente un atome métallique et R₁ représente un groupe alkyle inférieur de 1 à 4 atomes de carbone, contenant un alpha- ou beta-hydroxy acide, pour utilisation à titre de médicament dans le traitement et la prévention des signes du vieillissement cutané, de l'héliodermite et de la kératose actinique par la technique de la thérapie photodynamique.

L'invention a aussi pour objet l'utilisation d'une composition dermatologique combinant la chlorophylle ou un dérivé et un alpha-hydroxy acide avec l'acide aminolévulinique (ALA) et/ou un agent dépigmentant.

Suivant une caractéristique principale de la présente invention, la composition contient de la chlorophylle ou un dérivé de chlorophylle tel que la chlorophylline, comme substance photosensibilisante.

La chlorophylle est un pigment que l'on trouve dans de nombreux végétaux, en particulier dans les chloroplastes des cellules végétales.

Dans la présente invention, on peut utiliser les diverses chlorophylles a, b, c et d, ainsi que la chlorophylline et leurs dérivés obtenus par hydrolyse.

La chlorophylle et ses dérivés utilisables dans l'invention peuvent être représentés par la formule générale (I) ci-après : 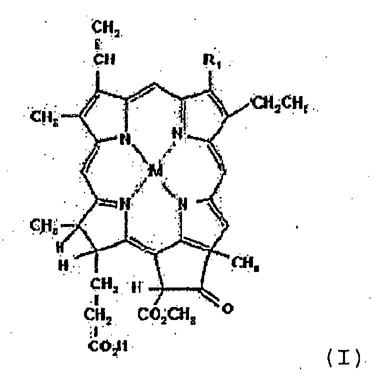 dans laquelle M représente un atome métallique et R₁ représente un groupe alkyle inférieur de 1 à 4 atomes de carbone, et de préférence un groupe méthyle -CH₃ (correspondant à la chlorophylle a) ou un groupe formyle -CHO (correspondant à la chlorophylle b).

L'atome de métal M est choisi parmi le magnésium, le cuivre, le cobalt, le nickel, le manganèse, le zinc et le fer. La chlorophylle est représentée par la formule (I) ci-dessus où M est un atome de magnésium. La chlorophylline utilisée dans la présente invention est représentée par la même formule (I) ci-dessus où M est un autre métal que le magnésium. La chlorophylline se distingue notamment de la chlorophylle par sa bonne solubilité dans l'eau tout en conservant sensiblement le même effet photosensibilisant.

Suivant une variante, outre le dérivé de chlorophylle, la composition de l'invention comprend de l'acide aminolévulinique, et, suivant une forme préférentielle de réalisation, elle comprend en association de la chlorophylline et de l'acide aminolévulinique. Les études efectuées ont montré en effet une action plus rapide et plus intense avec cette association par comparaison avec l'utilisation de la chlorophylline isolément.

La composition suivant l'invention est présentée sous forme de gel ou hydrogel réticulé, qui permet le relargage progressif des principes actifs et assure une bonne homogénéisation de la pénétration dans l'épiderme. L'hydrogel réticulé permet d'augmenter la pénétration trànsdermique du composé photosensibilisant tout en contrôlant son relargage progressif hors de l'hydrogel appliqué sur la peau. L'hydrogel est particulièrement bien adapté à la combinaison de chlorphylline et d'acide aminolévulinique, dont le taux de pénétration à travers la peau est alors amélioré. Il est également possible d'utiliser des techniques d'encapsulation ou de vectorisation connues pour assurer la libération régulée des principes actifs.

Cette composition est applicable sur la peau et est destinée à être irradiée par une source lumineuse de longueur d'onde comprise entre 610 et 650 nm, de préférence entre 625 et 635 nm, provoquant l'activation du composé photosensibilisant (chlorophylle, chlorophylline et dérivés). L'utilisation d'une source lumineuse à cette longueur d'onde permet d'agir avec une pénétration satisfaisante de l'agent photosensibilisant, de l'ordre de 5 à 8 mm environ, c'est-à-dire au niveau du derme.

Ce traitement est effectué de préférence après avoir effectué un premier peeling au moyen d'une composition à base d'alpha- ou beta-hydroxy-acide, par exemple l'acide glycolique, l'acide lactique, l'acide malique, l'acide tartrique, l'acide citrique, l'acide salicylique, et de préférence l'acide glycolique comme alpha-hydroxy acide et l'acide salicylique comme beta-hydroxy acide. Le traitement peut aussi être effectué simultanément avec le peeling.

La composition comprend un alpha- ou beta-hydroxy acide, en plus du composé photosensibilisant. Il est ainsi possible d'effectuer un premier traitement de peeling en appliquant la composition sur la peau pendant que le composé photosensibilisant migre progressivement dans l'épiderme et le derme, puis on procède à l'activation du composé photosensibilisant en exposant la peau à la lumière de longueur d'onde appropriée.

Il peut être avantageux, suivant une variante de réalisation de l'invention, d'incorporer dans la composition un ou plusieurs composés ayant une activité complémentaire, et par exemple un agent dépigmentant tel que l'arbutine, la glabridine, la vitamine C (acide ascorbique) ou l'acide kojique. Les essais effectués ont montré que l'effet dépigmentant se trouve potentialisé par l'utilisation de la composition de l'invention dans le cadre d'une technique de thérapie photodynamique. Le traitement peut alors être particulièrement efficace contre les tâches de pigmentation intense, résultant par exemple d'un traitement dermatologique au laser.

Suivant une forme avantageuse de réalisation, la composition à base de chlorophylle, ou de chlorophylline, est associée à un dépigmentant tel que l'acide kojique ou un de ses esters tels que le dipalmitate, l'arbutine ou un de ses dérivés, ou encore la glabridine.

Il peut aussi être avantageux d'incorporer un actif complémentaire tel que la diosgénine, ou un ester de diosgénine, utilisée pour son action adaptagène favorisant l'induction du métabolisme cellulaire pour rétablir l'homéostasie et pour son pouvoir antiradicalaire au niveau de l'action superoxyde, permettant de contrôler le taux de radicaux libres induits par l'action de la PDT.

Un principe actif complémentaire utilisable dans les compositions de l'invention, peut aussi être un anti-oxydant puissant tel que la super-oxyde dismutase, l'acide phytique, le coenzyme Q10, la diosgenine. Il est également avantageux d'ajouter à la composition un glycosaminoglycane hydrolysé.

Suivant une variante de réalisation de l'invention, on peut prévoir deux compositions appliquées successivement, la première contenant un principe actif complémentaire, tandis que la seconde contient le composé photosensibilisant.

Comme indiqué ci-dessus, le composé photosensibilisant utilisé dans l'invention est la chlorophylle, notamment la chlorophylle a ou la chlorophylle b, ou, de préférence, la chlorophylline, et plus particulièrement la 5-CR-chlorophylline®.

Le traitement au moyen de la composition de l'invention a pour avantage de stimuler l'activité fibroblastique et la synthèse de collagène et d'élastine dans le derme, entraînant ainsi une restructuration cutanée visible en général dès la 3^{ème} ou 4^{ème} séance de traitement. La durée de chaque séance de traitement dépend du diagnostic du praticien et de l'état du patient, et d'une manière générale elle peut être comprise entre 10 et 30 minutes environ.

L'effet de rajeunissement de la peau résulte de l'action des radicaux libres provenant de la photosensibilisation, qui provoquent une réaction inflammatoire et un processus de cicatrisation tissulaire sans brûlure. Elle se manifeste par une contraction de la peau qui entraîne une diminution des rides et ridules, et améliore l'ovale du visage.

Les essais effectués avec des compositions conformes à la présente invention ont mis en évidence une augmentation de la volémie, c'est-à-dire du volume sanguin total, et en particulier de la microcirculation, et du taux d'oxygène dans le tissu épidermique.

Les compositions suivant l'invention, en fonction des principes actifs complémentaires, peuvent être utilisées efficacement pour traiter des affections dermatologiques telles que le psoriasis, l'acné, le vitiligo ou le mélasma, avec dans ce dernier cas une absence de récidive, tant sur peau claire que sur peau foncée.

Les exemples suivants illustrent l'invention sans en limiter la portée. Dans ces exemples, les parties et pourcentages sont indiqués en poids, sauf indication contraire.

### Exemple 1

On prépare par des méthodes usuelles les formulations photosensibilisantes pour traitement par PDT indiquées ci-après.

**Formulation A**

| | |
|---|---|
| Gomme xanthane | 0,5 |
| Chitosan | 0,4 |
| Chlorophylline | 10,0 |
| Diosgenine | 1,0 |
| Conservateur | 1,0 |
| Eau | q.s.p. 100,0 |

**Formulation B**

| | |
|---|---|
| Gomme xanthane | 0,5 |
| Chitosan | 0,4 |
| Chlorophylline | 5,0 |
| Acide salicylique | 10,0 |
| Diosgenine | 1,0 |
| Conservateur | 1,0 |
| Eau | q.s.p. 100,0 |

**Formulation C**

| | |
|---|---|
| Gomme xanthane | 0,5 |
| Chitosan | 0,4 |
| Chlorophylline | 10,0 |
| Acide salicylique | 10,0 |
| Acide kojique | 5,0 |
| Acide phytique | 3,0 |
| Conservateur | 1,0 |
| Eau | q.s.p. 100,0 |

**Formulation D**

| | |
|---|---|
| Acide glycolique | 1,0 |
| Poly(méthacrylate de méthyle) | 2,5 |
| Propylène glycol dicaprylate/dicaprate | 3,5 |
| Stéarate de glycéryle | 1,0 |
| Acide oléique | 0,8 |
| Polysorbate 60 | 1,0 |
| PEG-100 stéarate | 0,8 |
| Ubiquinone | 0,8 |
| Gomme xanthane | 0,5 |
| Chlorophylline | 2,5 |
| Conservateur | 0,5 |
| Eau | q.s.p. 100,0 |

**Formulation E**

| | |
|---|---|
| Acide aminolévulinique | 1,0 |
| Chlorophylline | 8,0 |
| Gomme xanthane | 0,5 |
| Chitosan | 0,5 |
| Diosgenine | 1,0 |
| Conservateur | 0,5 |
| Eau | q.s.p. 100,0 |

### Exemple 2

### Etude clinique

Une étude a été faite sur 20 femmes âgées de plus de 50 ans présentant une héliodermite prononcée et des kératoses actiniques ou des rides au niveau du visage et du cou.

La durée totale de l'étude a été de 9 semaines, consistant à appliquer le traitement par PDT avec la composition conforme à la formulation D de l'Exemple 1, en exposant les zones à traiter pendant 20 minutes (longueur d'onde utilisée : 633 nm).

Chaque application de la composition est faite de la manière suivante.

La zone à traiter (visage et éventuellement cou) est nettoyée à l'aide d'un produit non alcoolisé et rincée à l'eau puis séchée. La composition de l'invention est ensuite appliquée (hydrogel réticulé et activateur) et on laisse reposer pendant environ 20 minutes.

On procède ensuite à l'irradiation (lampe Omnilux Rovivo 633 nm) pendant 20 minutes, en protégeant les yeux de la patiente par un masque adapté.

On applique ensuite une crème apaisante sur toute la zone traitée.

L'étude des cas traités a montré une nette amélioration de l'état de chaque patiente, dès la 3^{ème} application, sans séquelle cicatricielle.

## Revendications

1. Composition dermatologique sous forme de gel ou d'hydrogel réticulé, comprenant comme substance photosensibilisante de la chlorophylle ou un dérivé représentés par la formule générale (I) : 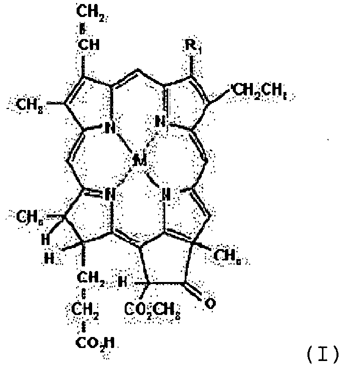 dans laquelle M représente un atome métallique et R₁ représente un groupe alkyle inférieur de 1 à 4 atomes de carbone, contenant un alpha- ou beta-hydroxy acide, pour utilisation à titre de médicament dans le traitement et
la prévention des signes du vieillissement cutané, de l'héliodermite et de la kératose actinique par la technique de la thérapie photodynamique.

2. Composition pour l'utilisation selon la revendication 1, **caractérisée en ce que** dans la formule générale (I) R₁ représente un groupe méthyle -CH₃ (correspondant à la chlorophylle a) ou un groupe formyle.

3. Composition pour l'utilisation selon la revendication 1 ou 2, **caractérisée en ce que** M est un atome métallique choisi parmi le magnésium, le cuivre, le cobalt, le nickel,
le manganèse, le zinc et le fer.

4. Composition pour l'utilisation selon la revendication 1, **caractérisée en ce que** la substance photosensibilisante est choisie parmi la chlorophylle a, la chlorophylle b, la chlorophylline et leurs dérivés obtenus par hydrolyse.

5. Composition pour l'utilisation selon l'une quelconque des revendications 1 à 4, **caractérisée en ce qu'**elle contient en outre de l'acide aminolévulinique.

6. Composition pour l'utilisation selon la revendication 5, **caractérisée en ce qu'**elle comprend en combinaison de la chlorophylline et de l'acide aminolévulinique.

7. Composition pour l'utilisation selon l'une quelconque des revendications 1 à 6, **caractérisée en ce qu'**elle contient en outre de la diosgénine, ou un ester de diosgénine.

8. Composition pour l'utilisation selon la revendication 1, **caractérisée en ce que** l'alpha-hydroxy acide est l'acide glycolique et le beta-hydroxy acide est l'acide salicylique.

9. Composition pour l'utilisation selon l'une quelconque des revendications 1 à 8, **caractérisée en ce qu'**elle contient en outre un agent dépigmentant ou éclaircissant, et/ou un anti-oxydant.

## Patentansprüche

1. Dermatologische Zusammensetzung in Form von Gel oder vernetztem Hydrogel, umfassend als photosensibilisierende Substanz Chlorophyll oder ein Derivat, dargestellt von der allgemeinen Formel (I): 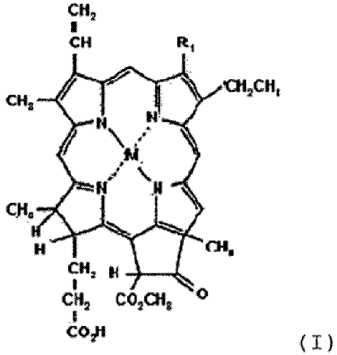 wobei M ein Metallatom darstellt und R₁ eine niedrige Alkylgruppe mit 1 bis 4 Kohlenstoffatomen darstellt, enthaltend eine Alpha- oder Betahydroxysäure, für die Verwendung als Arzneimittel bei der Behandlung und Vorbeugung von Alterungserscheinungen der Haut, der Sonnendermatitis und der aktinischen Keratose mittels der Technik der photodynamischen Therapie.

2. Zusammensetzung für die Verwendung nach Anspruch 1, **dadurch gekennzeichnet, dass** in der allgemeinen Formel (I) R₁ eine Methylgruppe -CH₃ (entsprechend dem Chlorophyll a) oder eine Formylgruppe darstellt.

3. Zusammensetzung für die Verwendung nach Anspruch 1 oder 2, **dadurch gekennzeichnet, dass** M ein Metallatom ist, ausgewählt aus dem Magnesium, dem Kupfer, dem Kobalt, dem Nickel, dem Mangan, dem Zink und dem Eisen.

4. Zusammensetzung für die Verwendung nach Anspruch 1, **dadurch gekennzeichnet, dass** die photosensibilisierende Substanz aus dem Chlorophyll a, dem Chlorophyll b, dem Chlorophyllin und ihren durch Hydrolyse gewonnenen Derivaten ausgewählt ist.

5. Zusammensetzung für die Verwendung nach einem der Ansprüche 1 bis 4, **dadurch gekennzeichnet, dass** sie ferner Aminolävulinsäure enthält.

6. Zusammensetzung für die Verwendung nach Anspruch 5, **dadurch gekennzeichnet, dass** sie in Kombination Chlorophyllin und Aminolävulinsäure enthält.

7. Zusammensetzung für die Verwendung nach einem der Ansprüche 1 bis 6, **dadurch gekennzeichnet, dass** sie ferner Diosgenin oder einen Diosgeninester enthält.

8. Zusammensetzung für die Verwendung nach Anspruch 1, **dadurch gekennzeichnet, dass** die Alphahydroxysäure die Glykolsäure ist und die Betahydroxysäure die Salicylsäure ist.

9. Zusammensetzung für die Verwendung nach einem der Ansprüche 1 bis 8, **dadurch gekennzeichnet, dass** sie ferner einen depigmentierenden oder aufhellenden Wirkstoff und/oder ein Antioxidans enthält.

## Claims

1. A dermatological composition as a cross-linked gel or hydrogel, comprising as a photosensitizing substance chlorophyll or a derivative represented by the general formula (I): 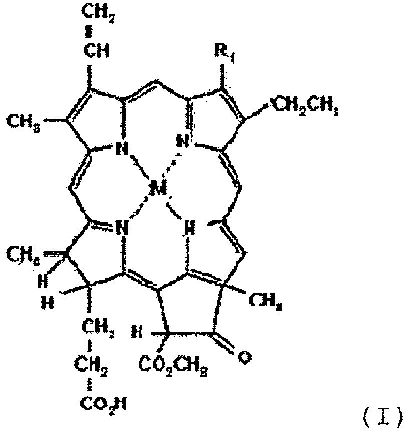 wherein M represents a metal atom and R₁ represents a lower alkyl group with 1 to 4 carbon atoms, containing an alpha- or beta-hydroxyacid, for use as a drug in the treatment and prevention of signs of skin ageing of heliodermitis and of actinic keratos is by the technique of photodynamic therapy.

2. The composition for the use according to claim 1, **characterized in that** in the general formula (1), R₁ represents a methyl group CH₃, corresponding to chlorophyll a) or a formyl group.

3. The composition for the use according to claim 1 or 2, **characterized in that** M is a metal atom selected from among magnesium, copper, cobalt, nickel, manganese, zinc and iron.

4. The composition for the use according to claim 1, **characterized in that** the photosensitizing substance is selected from among chlorophyll a, chlorophyll b, chlorophyllin and derivatives thereof obtained by hydrolysis.

5. The composition for the use according to any of claims 1 to 4, **characterized in that** it further contains aminolevulinic acid.

6. The composition for the use according to claim 5, **characterized in that** it contains a combination of chlorophyllin and of aminolevulinic acid.

7. The composition for the use according to any of claims 1 to 6, **characterized in that** it further contains diosgenin or a diosgenin ester.

8. The composition for the use according to claim 1, **characterized in that** the alpha-hydroxyacid is glycolic acid and the beta-hydroxyacid is salicylic acid.

9. The composition for the use according to any of claims 1 to 8, **characterized in that** it further contains a depigmenting or lightening agent and/or an anti-oxidant.
